# EUROPEAN PATENT APPLICATION

(11) **EP 1 216 674 A1**
(43) Date of publication of application: **26.06.2002**
(21) Application number: 00127794.6
(22) Date of filing: 19.12.2000
(51) Int. Cl.: A61F 13/42

(54) **Indicator means for absorbent products**

(71) Applicant: SCA Hygiene Products AB, 405 03 Göteborg (SE)
(72) Inventor: Kling, Robert, 511 63 Skene (SE)
(74) Representative: Harrison, Michael Charles

(57) **Abstract**

An indicator (13) for an absorbent product (1) provides an indication of the detected pH value. In order to detect potentially harmful conditions of pH within the product, the indicator means are arranged to selectively indicate pH values of 6.5 and above. In preferred embodiments, these indicator means may selectively indicate pH values of 6.7, 6.9, 7.1, 7.3 or 7.5 and above. A terpolymer or other indicator may be used as the selective pH indicator.

## Description

### Field of the invention:

The present invention relates to an indicator means for an absorbent product, wherein said absorbent product is of the type intended for the absorption of bodily exudates. In particular, the invention relates to pH indicator means for use in diapers or other absorbent garments.

### Background to the invention:

It is known from US-A-4 197 849 that absorbent pads may be provided with a pH indicating means in the form of a viewable, chemically reactive strip such as litmus paper which provides an indication of the pH of the patient's urine. In this way medical personnel can have an indication of desired urine characteristics.

Similarly it is known from US-A-5 217 444 that absorbent pads may be provided with one or more pH indicator materials in order to indicate the pH of secretions coming into contact therewith and thereby determine the wearer's health. In particular, it is disclosed that in order to predict conditions indicating the possibility of bacterial vaginosis or trichimonas vaginalis from vaginal discharge, an indicator can be used in a tampon to detect pH values of greater than 5.0, since normal vaginal discharge should have a pH less than about 4.5.

Document US-A-4 231 370 discloses the use of a pH indicator in absorbent products, whereby the presence of wetness by urine can be detected. In particular, the use of a pH-sensitive coating is proposed, by means of which urine can cause a change in colour of the indicator. When urine of pH 6.2 was used with a specific indicator, a colour change was observed within about 5 minutes. Lower pH values would however give a slower change whilst higher pH values would give a faster change. Thus, wetness at all normal pH values would be detected, merely with differing speed. Urine is disclosed as having a pH varying between 4.6 to 8.0, whilst having an average pH of about 6.0.

The soiling of diapers and other absorbent products is perhaps most commonly due to the presence of urine and/or faeces in such articles. In particular when the presence of faeces is detected, the product should be removed and replaced by a clean one. The soiled product may be discarded if it is the disposable type, or be cleaned and used again if it is of the reusable type.

With infants, diapers are changed typically between four and eight times during a twenty-four hour period. With adults, the frequency of changing incontinence garments such as diapers is often left to the adult's discretion based on a level of comfort or convenience, although when the wearer is bedridden, changing frequency depends on the attendant personnel. Diapers in particular are often not changed at an appropriate time when viewed in terms of the damage that may occur to a wearer's skin not only by the presence of urine alone, but in particular when urine and faeces are together in the diaper. This condition may often remain undetected for several hours, especially when the wearer exhibits no immediate signs of discomfort.

With babies for example, whose skin is particularly sensitive, diapers are often changed when the baby cries or otherwise indicates discomfort, or upon specifically checking the diaper for the presence of excess wetness and/or faeces. The checking process itself may often be misleading however, since an attendant often relies on an inspection by stretching open one leg elastic only, such that some faecal and urine discharges often go undetected.

Whilst urine is typically quickly absorbed and often held separate from the wearer's skin in an absorbent core (at least when no faeces is present), faeces is generally maintained against the skin since it does not pass through into the core, unless special provision is made for this in the top sheet design. When faeces is present first and then followed by urine, the urine itself may also be held in contact with the skin by the faeces. The presence of urine and faeces together is however recognised by the present inventors to be particularly harmful to the skin and a problem which should be solved.

This contact with the skin, and the presence of urine and faeces together, is harmful since this combined presence results in a chain of events causing breakdown of the skin. Faecal bacteria namely have urease which will react with urea from urine to thereby create ammonia. The presence of ammonia in the diaper will, in turn, raise the pH value and cause faecal protease and lipase to become more active, this activity increasing further with increasing pH. It is this activity which contributes to the wearer's skin becoming progressively and cumulatively damaged and leaves it at far greater risk to infections.

As will be apparent from the above, this is one of the reasons why nappy rash (diaper rash) and skin infections occur in body areas covered by absorbent products. Thus, whilst visual checks are made on the wearers of such products at specific or random intervals, the products in some cases (e.g. when simply wet) may in fact be less harmful even when left for long periods as compared to when urine and faeces are present together even for only relatively short periods. As in the aforementioned US-A-4 231 370, wetness may be used to indicate the need for a diaper change, although the indication of wetness by itself does not immediately result in damage to the skin, in particular when the urine has been stored in the absorbent core of the product, below a top sheet liner for example, away from the skin.

The present invention is thus aimed at providing a solution which helps further reduce skin damage, based on the recognition of harmful conditions within an absorbent product, to thereby allow said product to be changed when these conditions arise.

### Summary of the invention

The main objective is achieved by an absorbent product having the features defined in claim 1. Preferred features of the invention are defined in the dependent claims.

Further features of the invention will be apparent to the reader of this specification.

The present inventors have recognised a need to indicate the presence of conditions which are favourable to the increase of skin damage and in particular conditions which show a high probability for the presence of faecal bacterial activity in combination with urine.

In particular, it has been noted that whilst the pH of urine has an average of about 6.0, the generation of ammonia, caused by the presence of faeces-based urease for example, will cause the pH of urine to increase, and reach a value of about 6.0 or more. More certainty is provided by selecting the level of pH to be 6.5 and above or even higher selected values.

Similarly, even if a wearer has urine with a pH of 6.5 or more and even 6.7 or 6.9, 7.1, 7.3 or 7.5 or more, this should be indicated by itself since if faeces is released at this pH value in the absorbent product, the skin attack speed will be relatively high.

However, below pH 6.5, it is desirable to avoid an indication of harmful conditions being made in the indicator portion. Thus, in the present invention, the indicator means selectively indicates pH values of 6.5 and above, such that values below about 6.5 are not indicated. In a preferred embodiment, pH values of 6.7, 6.9, 7.1, 7.3 or 7.5 and above respectively may be selectively indicated.

However, it should be understood that an additional wetness indicator indicating values below 6.5, or 6.7, 6.9, 7.1, 7.3 or 7.5 and above respectively, may also be included in addition to the indicator means of the present invention. In this way, the additional indicator may indicate simply the condition of wetness as in the prior art, whilst the indicator means of the present invention would further indicate the existence of potentially harmful conditions.

It will be appreciated that the present invention has particular significance in absorbent products in which the exudates urine and faeces are typically present. Such products include especially diapers (for adult, infant and baby use), absorbent pants having an absorbent core (e.g. so-called training pants) as well as diaper chassis members and other incontinence products which have replaceable absorbent inserts. The invention may also find application in various other types of incontinence articles, such as sanitary napkins and the like.

### Brief description of the drawings

The invention will now be explained in more detail with reference to certain non-limiting embodiments thereof and with the aid of the accompanying drawings, in which:
- Fig. 1: shows a plan view of a diaper, with the liner removed,
- Fig. 2: shows a cross-sectional view onto line II-II of Fig. 1
- Fig. 3: shows a cross-section similar to that in Fig. 2 with a modified form of indicator
- Fig. 4: shows a schematic diagram illustrating an indicator means provided with a gate,
- Fig. 5: shows an indicator means with an encapsulated dye, and
- Fig. 6: shows a cross-sectional view through a diaper in the flat state depicting an indicator means with an external indicator portion.

### Detailed description of preferred embodiments:

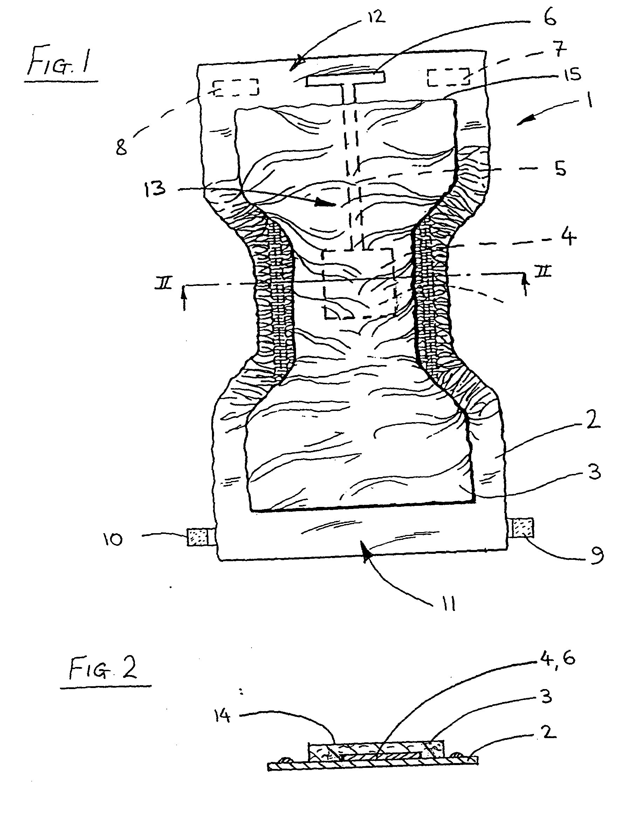

In Fig. 1, the absorbent product 1 in the form of a diaper has been illustrated in a flattened form and is depicted from the inner side thereof. For reasons of clarity, the top or cover sheet, sometimes referred to as a bodyside liner, has been removed.

Reference numeral 2 denotes a back sheet of liquid-impermeable material, typically consisting of a semitransparent PE film. Such back sheet materials are common in the field of current day diapers and allow the underlying elements to be discerned to a certain degree.

An absorbent core 3 of, for example, cellulosic material such as wood pulp fibres or the like, preferably mixed with a superabsorbent polymer, typically in an hour glass form, is positioned between the top sheet (not shown) and the back sheet 2. The absorbent core may be enveloped by a thin pervious layer such as a non-woven sheet (not shown) in some cases. The top sheet and back sheet 2 are then sealed around their contacting peripheries, preferably by means of welding or adhesive, to thereby form an envelope enclosing said core 3 and, if present, its enveloping pervious layer. The core 3 thus normally has dimensions which are smaller in both the length and width directions of the article, as shown.

Attachment means, such as hook and loop type mechanical fastening means, are provided at respective waist ends 11 and 12 of the article, whereby end 12 is intended to be the front waist portion of the product 1 and end 11 the rear waist portion, when applied to the wearer. In such an arrangement the tabs 7 and 8 could for example form loop tabs which are placed on the outer side of the back sheet 2, whilst tabs 9 and 10 could be hook tabs designed to engage with respective tabs 7 and 8 after the product has been passed through the legs of a wearer and up to the waist for attachment around the waist as is normal in the art.

An indicator means is attached to e.g. the back sheet 2 of the diaper and is denoted generally by numeral 13. As is further shown in Fig. 2, the indicator means 13 has one end thereof placed in contact with the absorbent core 3 in a generally centrally located portion, typically the crotch portion. The opposite end of said indicator means is, in this embodiment, shown lying at the front waist region 12 of said product 1, outside the end edge 15 of the core. The entire indicator means 13 is thus positioned on the inner, core-facing side of the back sheet 2 in this particular embodiment. However, the portion of said means 13 which is within the core 3 may be situated away from the back sheet 2, for example at a location a few millimetres below the upper surface 14 of said core 3.

The indicator means 13 in the embodiment illustrated comprises a detector portion 4 and an indicator portion 6, joined by a coupling transport portion or connecting line 5. The detector is thus positioned in a part of the absorbent core most likely to be affected by exuded bodily fluids. A suitable location for the detector portion 4 is thus in the crotch portion of the absorbent product, close to the middle portion thereof (as shown). In this way, urine will have a high probability of reaching the detector 4.

The detector portion could of course be given other shapes and forms and be located at another other part of the core or even at several different parts of the absorbent core, to provide more probability of urine reaching the detector portion 4. In a preferred embodiment, the detector portion 4 may be positioned between the topsheet and the absorbent core, i.e. on top of the core on the side closest to the wearer, where urine and faeces are mixed together.

The detector portion 4 is shown as being something different to the indicator portion 6 and spaced therefrom, although it should be understood that the detector portion may itself also fulfil the function of the indicator portion in certain embodiments, thus requiring no separate indicator portion, whereby the indicator portion and detector portion are the same thing.

The indicator portion 6 is intended to comprise any type of means that can provide a detectable indication, such as a visual indication, an audible indication, an indication by means of a specific fragrance, or even an indication by means of change of shape (e.g. for detection of a change of shape by blind persons), or even a combination of these indications.

In a preferred embodiment, in view of providing an indicator means which is cheap and can thus be used for mass production in disposable absorbent products, a visual indication is deemed most preferable. Thus in the embodiment of Fig. 1 the indicator portion 6 has been shown positioned in a relatively easily visible position on the diaper, approximately midway between the strips 7 and 8, where it is unlikely to be obscured by the fastening of strips 9 and 10 thereto. However, the indicator portion may be positioned at any suitable location, such as close to the crotch portion of the article, although it will be appreciated that such a location may make inspection of same somewhat more burdensome, although it would have the advantage of being cheaper and smaller overall than the indicator means shown in Fig. 1. Similarly the indicator means could have at least a part thereof positioned outside the wearer's normal clothing, in which case it would typically be attached by a physical communicating means to the detector portion inside the absorbent product, although it would be possible to construct the detector portion as a radio transmitter and the indicator portion as a receiver, thereby allowing the radio receiver portion to be a re-usable item.

When the indicator portion 6 and the detector portion 4 are spaced apart, a connection must be established therebetween. In the embodiment shown in Fig. 1 this is provided by a connector or transport means 5. The transport means 5 serves to activate the indicator portion 6 upon the detector portion 4 detecting a required parameter. In the present invention, the parameter to be detected is the pH value present in the fluid, normally urine, in an absorbent product.

In order to detect the presence of possibly harmful pH levels which correspond to those created when urine and faeces are present, the indicator means should detect, selectively, pH values of substantially 6.5 and above in the exudate and thus not be susceptible to values below about 6.5. In further embodiments, pH values of about 6.7, 6.9, 7.1, 7.3 or 7.5 and above respectively may be used as the detection threshold, since these are even more easily distinguishable from normal urine pH values which can be present dependent on the condition of the wearer, and a pH of above 7.0 is also a more certain and reliable indicator of the presence of urine and faeces together.

A cut-off of all pH values below exactly 6.5, or 7.0 respectively is not possible, but the window of pH values which will cause the indicator means to provide an indication can be closely controlled. Thus, for example, two or more visual indicator substances can be used to provide a detection of a small window of colour change ranges, whereby for instance a combination of phenol red and 3-nitrophenol could be used to detect whether a value of between 6.4 and 6.6 is present, as well as all values above 6.6. This can be arranged since the pH windows of these substances are about 6.4 to 8.2 and 6.6 to 8.6. Therefore, when phenol red has changed colour, but the 3-nitrophenol has not, the pH can be determined as lying between about 6.4 and about 6.6 whereas when phenol red and 3-nitrophenol have both changed colour, the value is clearly about 6.6 or above. Similar selections can be made for pH values of 7.0 and above by using the pH indicator windows of 4,5,6,7-tetrabromophenol, bromophenol red and alizarin for example.

Taking the example of a pH value of 6.5, the combination of the observed colour changes mentioned above for phenol and 3-nitrophenol can be used to indicate a pH value of about 6.5 and above.

A preferred type of selective detector/indicator envisaged by this invention is nevertheless a terpolymer. Terpolymers are used traditionally to provide enteric coatings for pharmaceutical compositions which can be broken down by intestinal fluids (see e.g. US-A-4 704 295). Terpolymers are also used in the field of detergent compositions for cleaning (see e.g. US-A-5 000 689). However it has now been appreciated by the inventors that terpolymers can be usefully employed in the present invention as indicators of pH. Terpolymers basically comprise three components; a temperature sensitive component, a polymer backbone (i.e. filler) and a pH sensitive component. In particular, terpolymers are formed by acid-ionisation such that they remain insoluble or impermeable at lower pH values, depending on the ionisation level. This feature is useable in the present invention, since the lowest pH at which the terpolymer dissolves or permeates can be arranged within fine limits to a specific pH value suitable for this invention. The techniques for fixing this pH value or set of values are readily known in the art of terpolymer construction and thus require no detailed description here.

In the embodiment of Figs. 1 and 2, the indicator portion 6 of the indicator means 13 may be constituted by a colour changing strip which is visible through the back sheet 2. As a further alternative, a highly transparent and fluid impervious window could be arranged in the back sheet. Although the embodiment of Fig.1 shows separate detector and indicator portions, the whole indicator means may be formed by a pH indicating strip.

A paper-based strip can be used to advantage since the capillary pressure in the paper will help to draw fluid from the core along the paper into the outer-lying portions of same, such as by drawing fluid from the core centre and up to the waistband.

This paper strip indication may also provide a measure of the amount of fluid in the absorbent product, depending on the capillary pressure in the paper and the distance travelled along the paper from the edge of the source of fluid. For example, if the diaper only contains a small amount of fluid, the fluid may not be sufficient to be drawn all the way from the fluid location in the absorbent core to the point of indication. However, when the amount of fluid is large, the paper strip will have plenty of fluid to draw upon in order for the fluid to reach the indicator portion of the indicator means. Thus, detections of only small amounts of urine can be avoided by appropriate material choice.

As a further alternative, the detector portion 4 can be arranged to cause activation of the indicator portion 6 by sending a signal of some type (e.g. a colour signal, electrical signal, fluid signal etc.) by means of a connector portion or line 5.

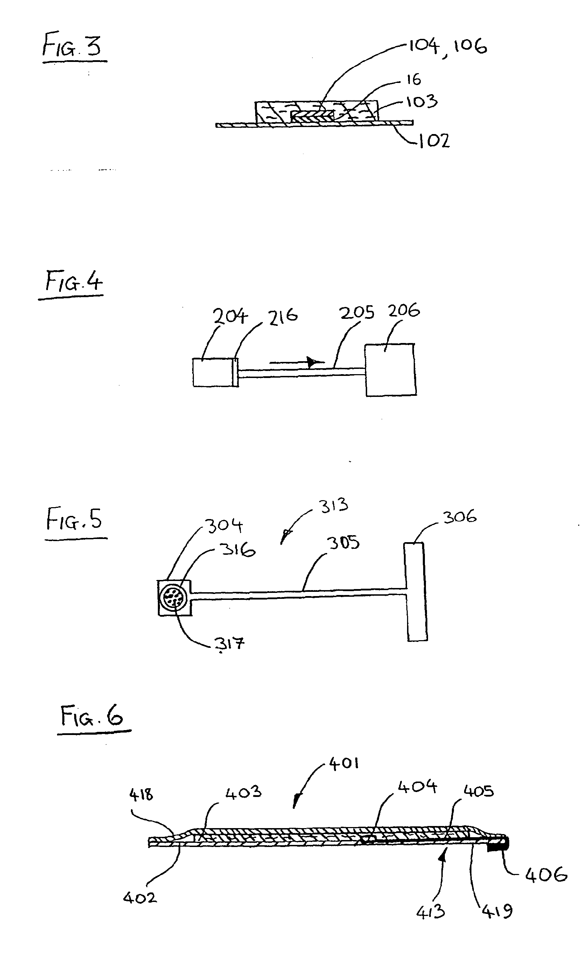

Fig. 3 shows a further embodiment of the indicator means 13, similar to the sectional view shown in Fig. 2, whereby like features have like reference numerals raised by 100. In Fig. 3 the indicator portion 106 (and commensurate detector portion 104, which may be the same thing in some embodiments) is separated from the back sheet by an intermediary covering layer 16 which is dissolvable at the required pH levels.

A terpolymer arranged to dissolve selectively at pH of about 6.5 and above or 6.7, 6.9, 7.1, 7.3 or 7.5 and above respectively could be used as a suitable covering layer material. When the covering layer 16 is itself the pH-sensitive means, the indicator portion 106 may then be an "inert" indicator, such as a strip of a permanent single bright fluorescent yellow colour, whereby when the covering layer 16 is dissolved or broken away or permeated, the fluorescent colour becomes clearly visible through the back sheet 102, but does not itself undergo any change.

Additionally to the possibility of the covering layer being an indicator, the indicator means 13 could itself also be colour changeable at or above the required pH, and protected by the covering layer (e.g. terpolymer). In this way, the covering layer 16 would provide a first barrier to the indicator portion 106 being visible and, when the covering layer is removed (e.g. the terpolymer dissolves or becomes permeable), the indicator could be allowed to come into contact with the substance having a pH of 6.5 or above and thus change colour. This could be used to provide a dual-level indicator system, whereby the covering layer (terpolymer) breaks down and dissolves at for example pH 6.5 to reveal the underlying indicator portion 106 in one colour, whereas when the pH rises to e.g. 7.0 or a higher value, the indicator can assume a further colour which is also visible. In this way, the wearer or attendant can be warned at a first level (caused by the terpolymer dissolving for example) that harmful conditions exist and then at a second level (caused by the indicator portion 6 colour change) that the pH has risen to a new higher level (e.g. 7.0 or even 8.0) such that rapid degradation of the skin is likely to occur unless the absorbent product is changed.

Although in Fig. 3, the covering layer (e.g. terpolymer) 16 is shown as being a coating on only one side of the indicator means 13 (i.e. the elements 104 and 106 in this case), the indicator means 13 (or any specific portion(s) thereof) may be entirely enclosed in the covering layer 16 on its entire surface. Similarly, the covering layer 16 is shown as being coextensive with the combined indicator and detector portions 106 and 104, but could of course be wider and longer than same so as to prevent the side portions of same being seen through the back sheet until required.

The thickness of the covering layer 16 depends on the desired time until visual indication should take place after the lowest selected pH level is reached. This may be used advantageously so as to avoid warning the wearer or attendant of a urine pH value which is possibly high and undesirable, but which at the same time is so small that it is entirely absorbed in the lower part of the core 3 (i.e. part closest to the back sheet 2) and thus of relatively insignificant danger for the wearer's skin.

Fig. 4 illustrates a sketch of a further principle whereby a blocking layer 216 (e.g. a terpolymer or other pH-sensitive substance) forms a block, or gate, preventing any signal produced by the detector 204 from reaching the connector or the signal transport means 205, to thereby inhibit the indicator portion 206 from being activated to produce an indication. For example, detector 204 may provide a source of coloured dye that is to be transported to indicator portion 206 by means of a line 205 (e.g. a capillary filled with water, or a series of capillaries filled with water), whereby the pH-sensitive blocker 216 first needs to be removed or opened to allow movement of the dye down the line 205.

Instead of a source of coloured dye, detector 204 may instead contain a first active substance or composition which, when released, can react with a second active substance or composition in the presence of liquid in the line 205 to produce a colour change visible in for example line 205 and/or indicator portion 206. Since there are many possibilities of chemicals which can produce such a colour change which are known to the skilled person, further discussion of same here is unnecessary.

Similarly, line 205 could be an electrical conductor transferring a current generated by detector portion 204 as a result of pH level being reached, whereby blocker 216 is in the form of a switch preventing current flow below a certain threshold, but when removed or opened allows a current flow to activate indicator portion 206 (e.g. by an audible and/or visual indication). Detector portion 204 and blocker 216 can of course be an integral unit or in fact the same thing. Fig. 4 is thus merely illustrative of the principle of operation and is not intended to dictate the positional interrelationship of the various elements. Preferably, a terpolymer blocker is applied as a coating over the detector portion.

Fig. 5 shows an embodiment similar to Fig. 4, whereby a coloured dye 317 is stored in an encapsulation of a pH sensitive blocker material 316 as part of detector portion 304, whereby upon breakdown of the blocker material 316 (e.g. a terpolymer), the dye is allowed to travel along path 305 to be displayed by the indicator portion 306. The detector portion 304 and the connector line 305 can thus be made of an opaque material, whilst leaving the indicator portion 306 transparent. Alternatively, the entire indicator means 313 could have a single base material which is stainable all over when the dye 317 is released.

As an alternative (not shown), the dye 317 might be replaced by, or supplemented with, a foam substance which when activated due to the pH level obtained, causes foam to be formed at a fast rate and thereby fill an expandable cavity in an indicator means 306 (or in portion 304 if portions 304 and 306 are combined at the same location). In this way, a change of shape can constitute the indication, whereby the indicator means 306 would be placed in a location where the change of shape could be easily felt and/or seen by the user. This may be particularly suitable for users who are colour-blind or have defective sight. For example, the indicator means may provide a smooth surface having weakened expandable portions in the normal state, whereby filling the indicator means 306 with foam or gas or the like (as a result of pH above 6.5) would expand said weakened portions and thus change the texture of the smooth surface so as to be recognisably different to the surface in its original state.

Fig. 6 shows a further embodiment of the invention, in which an indicator means 413 having a detector portion 404, connector line 405 and indicator portion 406, is arranged such that the indicator portion 406 lies on or adjacent to the external surface 418 of the back sheet 402, whilst the detector portion 404 and part of line 405 may be arranged between the top sheet 418 and back sheet 402, the detector portion being in or adjacent to the absorbent core 403. In this way, perception of the indicator portion is facilitated, be it audio, visual or a combination of any number of perceivable indications.

The blocking layer 216 or the blocking material 316 may take many forms and although a terpolymer has been proposed in a preferred embodiment, other possibilities exist. The basic principle is however that the layer or material must dissolve in mildly alkaline conditions, but not at about below pH 6.5.

A further preferred embodiment of the layer of material is for example a pH sensitive hydrogel. Carboxylated hydrogels are particularly suitable since they are non-toxic, cheap and present a sharp pH shift, thus allowing high accuracy pH detection in the present invention. pH-sensitive hydrogels swell in such a way that they become permeable to both small and large molecules. The critical value of pH at which the hydrogel will swell is dependent on the pKa value of the acid moieties of the hydrogel. In this way, the skilled person can provide a pH value adjusted to swell at the required pH. Thus, taking the embodiment of Fig. 4 for example, layer 216 which forms the gate or blocker described above can be formed by a hydrogel which initially is plugged by large molecules which are freed, at the required pH, due to the swelling of the hydrogel. The freeing of the large molecules opens one or more channels through which the contents of part 204 comes into contact with the contents of part 205. This can likewise be applied to the embodiment of Fig. 5 by construction of the layer 316 as a hydrogel carrying large molecules which upon swelling are freed to open channels allowing dye 317 (or other component) to pass through the layer 316.

A further alternative is the use of any one of many known mildly acidic salts which will saturate at low pH levels and dissolve freely in alkali conditions. Depending on the pH dissolution level required, it is merely a matter of selecting a salt e.g. from the Merck Index listing of strong acids and weak bases (e.g. calcium sulphate salt or the like). This selection is not described in further detail here since the selection of specific salts is not a limitation of the present invention. It may also be mentioned that there are also any number of low-molecular weight organic acid salts which are readily available having a wide range of dissolution points.

A still further possibility is the use of chitosan as the blocking material or layer. Chitosan is namely soluble in dilute aqueous organic acids (thus including values for example pH values between 6.5 to 7.0) and thus applicable for use in the invention. Chitosan is namely not soluble in many other solutions.

Although dissolution of the gate of blocking material or layer upon the required pH value being reached has been described in some of the above embodiments, further possibilities include constructing the gate from a compound which exhibits a marked change in viscosity at a particular level of pH. For example, proteins have no significant shift in solubility as pH varies since they are highly amphoteric. However, proteins do show a shift in viscosity value or gel point with pH which can be sufficient for this invention. For example, taking the embodiment of Fig. 4, the line 205 may be constructed as a capillary filled with fluid, which capillary can be plugged with a highly viscous polymeric gel which becomes less viscous at the required pH (e.g. at pH 6.5 and above, 6.7 etc). This then allows the plug to be freed at the required pH. The selection of the requisite polymeric gel is not described in further detail here, since the specific polymer can simply be selected according to the pH value required.

Further embodiments will be readily understood by the skilled person upon reading the aforegoing and are intended to be encompassed within the scope of the invention as defined by the appended claims.

## Claims

1. An indicator means (13; 313; 413) for an absorbent product (1; 401), wherein said indicator means (13; 313; 413) provides an indication of the detected pH value in said absorbent product (1; 401), **characterized in that** said indicator means (13; 313; 413) selectively indicates pH values of 6.5 and above.

2. An indicator means according to claim 1, **characterized in that** said indicator means selectively indicates pH values of 6.7 and above.

3. An indicator means according to claim 1 or 2, **characterized in that** said indicator means selectively indicates pH values of 6.9 and above.

4. An indicator means according to any one of the preceding claims, **characterized in that** said indicator means selectively indicates pH values of 7.1 and above.

5. An indicator means according to any one of the preceding claims, **characterized in that** said indicator means selectively indicates pH values of 7.3 and above.

6. An indicator means according to any one of the preceding claims, **characterized in that** said indicator means selectively indicates pH values of 7.5 and above.

7. An indicator means according to any one of the preceding claims, **characterized in that** said indicator means provides a visual and/or audible indication.

8. An indicator means according to any one of the preceding claims, **characterized in that** said indicator means comprises a detector portion (4; 104; 204; 304; 404) and an indicator portion (6; 106; 206; 306; 406).

9. An indicator means according to claim 8, **characterized in that** said detector portion (4; 104; 204; 304; 404) is spaced from said indicator portion (6; 106; 206; 306; 406) to allow detection at a different location to indication.

10. An indicator means according claim 8, **characterized in that** said detector portion (4; 104; 204; 304; 404) comprises a pH-sensitive detector, and **in that** said detector portion (4; 104; 204; 304; 404) causes activation of said indicator portion (6; 106; 206; 306; 406) when the pH value is within the selected pH range.

11. An indicator means according to claim 10, **characterized in that** said detector portion comprises a pH-sensitive terpolymer (16; 216; 316), said terpolymer preventing said indicator portion (6; 106; 206; 306; 406) being activated when said pH level is below the selected pH range, and being dissolvable within the selected pH range to thereby allow activation of said indicator portion (6; 106; 206; 306; 406).

12. An indicator means according to claim 11, **characterized in that** said terpolymer is applied as a coating over said indicator portion (106), whereby the terpolymer obscures the indicator portion from view until dissolved.

13. An indicator means according to claim 11, **characterized in that** said terpolymer (216; 316) is applied as a barrier interrupting the connection between said detector portion (204, 304) and said indicator portion (206; 306), whereby the terpolymer prevents the detector communicating with the indicator portion until dissolved.

14. An indicator means according to claim 13, **characterized in that** said terpolymer (216) is applied as a coating over said detector portion (204).

15. An indicator means according to any one of claims 8 to 14, **characterized in that** said indicator means (313) contains an encapsulated dye (317), which is released upon activation of said detector portion (304).

16. An indicator means according to any one of the preceding claims, **characterized in that** said indicator means (13; 313; 413) is attached to an absorbent product (1; 401).

17. An indicator means according to claim 16, **characterized in that** an indicator portion (406) of said indicator means (413) is positioned on an outer surface of said absorbent product (401).

18. An indicator means according to claim 16, **characterized in that** an indicator portion (6; 106) of said indicator means (13) is positioned on said absorbent product (1) at a location lying internally of an outer layer (2; 102) of said absorbent product but visible through said outer layer (2; 102).

19. An indicator means according to any one of claims 16 to 18, **characterized in that** a detector portion (404; 104; 404) of said indicator means is positioned in, or adjacent to, an absorbent part (3; 103; 403) of said absorbent product (1; 401).

20. An indicator means according to claim 19, **characterized in that** said detector portion (4) is positioned between a top sheet and said absorbent part (3).

21. An indicator means according to claim 10, **characterized in that** said detector portion comprises a pH-sensitive hydrogel, said hydrogel preventing said indicator portion (6; 106; 206; 306; 406) being activated when said pH level is below the selected pH range, and being caused to expand so as to provide at least one passageway therein, within the selected pH range, to thereby allow activation of said indicator portion (6; 106; 206; 306; 406).

22. An indicator means according to claim 10, **characterized in that** said detector portion comprises a pH-sensitive acidic salt, said salt preventing said indicator portion (6; 106; 206; 306; 406) being activated when said pH level is below the selected pH range, and being dissolvable within the selected pH range to thereby allow activation of said indicator portion (6; 106; 206; 306; 406).

23. An indicator means according to claim 10, **characterized in that** said detector portion comprises chitosan, which prevents said indicator portion (6; 106; 206; 306; 406) being activated when said pH level is below the selected pH range, and being dissolvable within the selected pH range to thereby allow activation of said indicator portion (6; 106; 206; 306; 406).

24. An indicator means according to claim 10, **characterized in that** said detector portion comprises a protein, said protein having a gel point selected to provide a high viscosity so as to prevent said indicator portion (6; 106; 206; 306; 406) being activated when said pH level is below the selected pH range, and exhibiting a viscosity decrease within the selected pH range to thereby allow activation of said indicator portion (6; 106; 206; 306; 406).
